# EUROPÄISCHE PATENTSCHRIFT

(11) **EP 4 486 716 B1**
(45) Veröffentlichungstag und Bekanntmachung des Hinweises auf die Patenterteilung: **09.09.2026**
(21) Anmeldenummer: 23710210.8
(22) Anmeldetag: 01.03.2023
(51) Int. Cl.: C07C 19/045, C07C 17/02, C02F 1/16, C02F 1/06, B01D 1/00, B01D 1/26, B01D 3/06, B01D 3/14

(54) **THERMISCHE KOPPLUNG EINER ANLAGE ZUR HERSTELLUNG VON 1,2-DICHLORETHAN MIT EINER ANLAGE ZUR THERMISCHEN ENTSALZUNG (VON MEERWASSER)**
THERMAL COUPLING OF A PLANT FOR PREPARING 1,2-DICHLOROETHANE TO A PLANT FOR THERMAL DESALINATION (OF SEA WATER)
COUPLAGE THERMIQUE D'UNE INSTALLATION DE PRODUCTION DE 1,2-DICHLOROÉTHANE AVEC UNE INSTALLATION DE DESSALEMENT THERMIQUE (DE L'EAU DE MER)

(30) Priorität: 02.03.2022 DE 102022104952
(43) Veröffentlichungstag der Anmeldung: 08.01.2025
(73) Patentinhaber: thyssenkrupp Uhde GmbH, 44141 Dortmund (DE); thyssenkrupp AG, 45143 Essen (DE); Westlake Vinnolit GmbH & Co. KG, 85737 Ismaning (DE)
(72) Erfinder: BENJE, Michael, 65182 Bad Soden (DE); KAMMERHOFER, Peter, 84508 Burgkirchen (DE); KREJCI, Klaus, 84489 Burghausen (DE)
(74) Vertreter: thyssenkrupp Intellectual Property GmbH
(86) Internationale Anmeldenummer: PCT/DE2023/100163
(87) Internationale Veröffentlichungsnummer: WO 2023/165656

(56) Entgegenhaltungen:
- EP-B1- 1 899 287
- WO-A1-2022/015879

## Beschreibung

Die Erfindung betrifft ein Verfahren zur Erzeugung von 1,2-Dichlorethan aus Ethylen und Chlor und zur Wasserentsalzung, wobei die jeweiligen Prozesse in thermisch miteinander gekoppelten Anlagenteilen durchgeführt werden. Die vorliegende Erfindung betrifft weiterhin zur Durchführung solcher Verfahren ausgelegte Anlagen und die Verwendung von Wärme aus einem Verfahren zur Erzeugung von 1,2-Dichlorethan aus Ethylen und Chlor zur Erhitzung von in einer Wasserentsalzung zu behandelndem Wasser.

### Stand der Technik

Polyvinylchlorid (PVC) stellt aufgrund seiner Haltbarkeit insbesondere gegenüber Sonnenlicht ein wichtiges Polymer für Anwendungen in langlebigen Produkten, z.B. im Außenbereich dar. Polyvinylchlorid wird dazu durch Polymerisation aus monomerem Vinylchlorid (VCM) hergestellt, das heute in der Regel über eine zweistufige Synthese aus Ethylen (CH₂=CH₂) und elementarem Chlor (Cl₂) erzeugt, wobei als Zwischenprodukt zunächst 1,2-Dichlorethan (CH₂Cl-CH₂Cl, auch als EDC bezeichnet) gebildet wird. Durch Abspaltung von Chlorwasserstoff (HCl) wird anschließend aus dem 1,2-Dichlorethan Vinylchlorid gebildet:

| | |
|---|---|
| Cl₂ + C₂H₄ → C₂H₄Cl₂ (Rein-EDC) | +180 kJ/Mol |
| C₂H₄Cl₂ (Spalt-EDC) → C₂H₃Cl (VCM) + HCl | -71 kJ/Mol |

Ein entsprechendes Verfahren zur Herstellung von 1,2-Dichlorethan aus Ethylen ist z.B. in der DE 19910964 A1 oder der DE 102008020386 A1 beschrieben, wobei das Produkt 1,2-Dichlorethan hier auch als Reaktionsmedium wirkt. Dieses Verfahrenskonzept ist auch als Direktchlorierung bekannt. Aus der angegebenen Reaktionsgleichung ist ersichtlich, dass die Reaktion von Ethylen mit Chlor zu 1,2-Dichlorethan stark exotherm ist.

Bei der Direktchlorierung unterscheidet man zwischen Kaltchlorierungs(LTDC)- und Heißchlorierungs-(HTDC)-Verfahren. Reaktionsmedium ist bei beiden Verfahrensvarianten das flüssige Reaktionsprodukt selbst, in dem ein Lewissäure- (meist Eisen-III-chlorid)-Katalysator gelöst ist.

Bei einem LTDC-Verfahren, das beispielsweise in US 2,393,367 A beschrieben ist, wird die Reaktion bei ca. 50-60°C durchgeführt. Die Reaktionswärme von ca. 2000 kJ/kg EDC wird dabei vollständig durch Kühlwasser oder aber einen/mehrere Luftkühler abgeführt. Durch die niedrige Reaktionstemperatur ist auch die Bildungsrate unerwünschter Nebenprodukte wie 1,1,2-Trichlorethan gering. Allerdings erlaubt die niedrige Reaktionstemperatur keinerlei Wärmenutzung bzw. - Rückgewinnung. Zudem muss das Reaktionsprodukt mit Wasser katalysatorfrei gewaschen werden, wobei wiederum Abwasser entsteht, das gesondert behandelt werden muss. Aufgrund dieser Nachteile haben LTDC-Verfahren stark an Bedeutung verloren.

Bei den HTDC-Verfahren wird die Reaktion in der Praxis meist bei Temperaturen zwischen 100 °C und 130 °C geführt. Das Reaktionsprodukt EDC wird dampfförmig aus dem Prozess abgezogen, wobei der gelöste Katalysator im Reaktor verbleibt. Ein solches Verfahren wird beispielsweise in der GB 1 554 658 beschrieben. Bei diesen Verfahren ist Wärmerückgewinnung möglich - beispielsweise kann das Reaktionsprodukt direkt dampfförmig in eine Kolonne zur destillativen Aufreinigung eingespeist werden oder zur indirekten Beheizung von Destillationskolonnen verwendet werden.

Die meisten HTDC-Verfahren werden als Reaktivabsorption geführt, d.h. der gasförmige Reaktionspartner Chlor wird zunächst in einem umlaufenden Strom des Reaktionsmediums EDC aufgelöst, wobei der umlaufende EDC-Strom durch Zwangsumlauf oder Naturumlauf in der Reaktionsanordnung zustande kommen kann. In dem umlaufenden, gelöstes Chlor enthaltenden EDC-Strom wird dann gasförmiges Ethylen eingespeist, das nach Durchtritt durch die Phasengrenzfläche mit dem gelösten Chlor reagiert.

Überraschenderweise zeigte sich, dass auch bei Reaktionstemperaturen von typischerweise 120° C ein qualitativ sehr hochwertiges Produkt erzeugt werden kann, wenn die Direktchlorierung homogen in der Flüssigphase durchgeführt wird.

Die Umsetzung erfolgt in der Praxis beispielsweise in einem Schlaufenreaktor mit einem Steigrohr, einer Verdampfungszone und einem Fallrohr, in dem die Reaktionsmischung im Naturumlauf (d.h. ohne dass in wesentlichen Umfang Strömungsenergie eingebracht wird) geführt wird. Das produzierte 1,2-Dichchlorethan wird aus der Verdampfungszone am oberen Ende des Steigrohres dampfförmig abgezogen.

Die treibende Kraft für den Naturumlauf (d.h. die Auftriebskraft im Steigrohr) wird durch die Zugabe des gasförmigen Reaktanden Ethylen im unteren Teil des Steigrohrs einerseits und durch die teilweise Verdampfung des umlaufenden 1,2-Dichlorethans im oberen Teil des Steigrohrs andererseits erzeugt. Beide Vorgänge führen zu einem gewissen Gasanteil und damit zu einer niedrigeren mittleren Dichte im Steigrohr als im Rest des Reaktors. Unterstützt wird der Naturumlauf zudem dadurch, dass die Temperatur des flüssigen 1,2-Dichlorethans im Steigrohr aufgrund der exothermen Umsetzung höher und damit dessen Dichte im Vergleich zu dem 1,2-Dichlorethans im Fallrohr geringer ist.

Die Umsetzungsreaktion erfolgt in flüssiger Phase, indem gasförmiges Ethylen im unteren Teil des Steigrohres über einen Verteiler gasförmig zugegeben wird und sich dann im aufwärts strömenden 1,2-Dichlorethan auflöst.

In der Praxis wird für das Einbringen des Chlors in das 1,2-Dichlorethan bei dem Verfahren nach DE 19910964 A1 bzw. DE 102008020386 A1 ein Seitenstrom von flüssigem 1,2-Dichlorethan, z.B. aus dem Fallrohr des Reaktors, entnommen und mittels mindestens eines Wärmeüberträgers abgekühlt. Das abgekühlte 1,2-Dichlorethan dient als Treibstrom in einem Flüssigkeitsstrahl-Gasverdichter (z.B. einer Injektordüse), mittels dessen gasfömiges Chlor angesaugt wird. Aufgrund der niedrigen Temperatur des Treibstromes und der dadurch bedingten guten Löslichkeit des Chlors in EDC liegt das Chlor schon am Austritt des Flüssigkeitsstrahl-Gasverdichters in 1,2-Dichlorethan vollständig gelöst vor. Diese Lösung wird dann dem Steigrohr des Reaktors über einen Flüssigkeitsverteiler wieder zugeführt, wo es mit dem gelösten Ethylen unter Bildung von 1,2-Dichlorethan reagiert.

Die Reaktionsführung in flüssiger Phase führt zu einer gegenüber anderen Verfahren deutlich verringerten Bildung von Nebenprodukten. Insbesondere werden bei Reaktionstemperaturen von typischerweise 120°C Produktreinheiten erzielt, für die bei anderen Verfahren zur Unterdrückung unerwünschter Nebenreaktionen wesentlich niedrigere Reaktionstemperaturen erforderlich sind.

Zur Temperaturkontrolle muss aus dem Prozess insgesamt Wärme abgeführt werden, die zur Beheizung von Wärmesenken in anderen Prozessen verwendet werden kann. So kann beispielsweise der 1,2-Dichlorethan-Brüden vom Kopf des Reaktors zur mantelseitigen Beheizung des Umlaufverdampfers einer Destillationskolonne verwendet werden oder direkt dampfförmig in eine Destillationskolonne zur weiteren Aufreinigung eingespeist werden, wobei die jeweils äquivalenten Dampfmengen, die sonst zur Kolonnenbeheizung erforderlich wären, eingespart werden.

Ebenso kann der zur Lösung von Chlor aus dem Reaktor abgezogene 1,2-Dichlorethan-Strom abgekühlt werden, indem beispielsweise der Umlaufverdampfer einer Destillationskolonne mantelseitig beheizt wird. Ein Verfahren, bei dem diese Methoden angewendet werden, wird z.B. in der EP 1 228 022 B1 beschrieben.

Mittels des dampfförmigen und des flüssigen 1,2-Dichlorethanstroms können auch weitere Wärmerückgewinnungsmaßnahmen realisiert werden. So beschreibt beispielsweise die EP 1 899 287 B1 ein Verfahren, bei dem die Reaktionswärme der Direktchlorierung zur Beheizung einer Anlage zur Eindampfung von Natronlauge aus einer Chlor-Alkali-Elektrolyseanlage verwendet wird.

Eine weitere Möglichkeit für die Verwendung der Reaktionswärme beschreibt die DE 10 2011 014 131 A1, in der die Reaktionswärme zur Trocknung eines feuchten Polymerpulvers (insbesondere PVC) genutzt wird.

Allen hier beschriebenen Verfahren ist gemeinsam, dass der fühlbare Wärmeinhalt des flüssigen 1,2-Dichlorethanstromes nur teilweise genutzt werden kann, da die fühlbare Wärme typischerweise oberhalb von ca. 95 °C abgeführt werden muss.

Um den Reaktanden Chlor in dem flüssigen 1,2-Dichlorethanstrom auflösen zu können, muss letzterer jedoch auf etwa 50 bis 60° C abgekühlt werden. Dies erfolgt in der Regel durch eine Wasserkühlung, so dass ein Teil der fühlbaren Wärme des 1,2-Dichlorethanstromes wegen des niedrigen Temperaturniveaus nicht genutzt werden kann. Insgesamt können daher im beschriebenen Stand der Technik nur etwa 60 % der Reaktionswärme der Direktchlorierung (ca. 600 kWh/t 1,2-Dichlorethan) durch Maßnahmen zur Wärmerückgewinnung genutzt werden. WO 2022/015879 A1 beschreibt ein hybrides Verfahren zur Abtrennung von Wasser aus einer Salzlösung, bei dem die Salzlösung einem ersten Trennprozess zugeführt wird, wodurch ein Wasserdestillat und eine konzentrierte Salzlösung erzeugt werden. Die konzentrierte Salzlösung wird einem zweiten Trennprozess zugeführt, der bei einer höheren Temperatur durchgeführt wird als der erste Trennprozess. Aus dem zweiten Trennprozess abgeführte Wärme wird für dem ersten Trennprozess zugeführt. Die Hochtemperaturwärme für den zweiten Trennungsprozess kann aus einer thermische Energiequelle stammen, die thermische Solarenergie, geothermische Energie, Kernenergie, eine exotherme chemische Reaktion oder eine Kombination davon umfassen kann.

Vor diesem Hintergrund besteht ein Bedarf für ein Verfahren, das eine weitergehende und möglichst vollständige Nutzung von bei der Direktchlorierung von Ethylen entstehender Wärme erlaubt. Die vorliegende Erfindung befasst sich mit diesem Bedarf.

### Beschreibung der Erfindung

Die Erfinder haben überraschend festgestellt, dass eine weitgehende Nutzung der bei der Direktchlorierung von Ethylen anfallenden Wärme für eine Wasserentsalzung genutzt werden kann. Dies ist insbesondere für Standorte von großem Vorteil, wo Salzwasser, z.B. als Meerwasser, in großen Mengen vorhanden ist, aber niederschlagsbedingt kaum Süßwasser verfügbar ist. Entsprechend erzeugtes entsalztes Wasser kann z.B. für eine Chloralkalielektrolyse verwendet werden, in der das für die Direktchlorierung benötigte Chlor hergestellt wird.

Demzufolge betrifft die vorliegende Erfindung in einem ersten Aspekt ein Verfahren zur Erzeugung von 1,2-Dichlorethan aus Ethylen und Chlor und zur Wasserentsalzung, wobei in einem ersten Anlagenteil Ethylen mit Chlor zu 1,2-Dichlorethan umgesetzt und in einem zweiten Anlagenteil eine Wasserentsalzung durchgeführt wird, dadurch gekennzeichnet, dass die bei der Umsetzung von Ethylen mit Chlor entstehende Wärme für eine Erhitzung von Wasser in der Entsalzung genutzt wird, wobei die Wasserentsalzung als Multistage Flash Evaporation oder als Multieffekt-Destillation, bevorzugt als Multieffekt-Destillation, durchgeführt wird, wobei Wasser als Wärmetransfermedium für die Übertragung der Wärme von ersten zum zweiten Anlagenteil genutzt wird und wobei das Wasser zum Abkühlen des 1,2-Dichlorethans auf maximal 95°C erwärmt wird.

Bei der Wasserentsalzung handelt es sich bevorzugt um eine Entsalzung von Wasser mit einem Salzgehalt von 2 bis 5 Gew.-% und insbesondere 2,8 bis 3,8 Gew.-%. Ganz besonders bevorzugt wird Wasser mit einem Salzgehalt von etwa 3,5 Gew.-% entsalzt, d.h. insbesondere Meerwasser.

Die Art und Weise, wie die Wasserentsalzung durchgeführt wird, ist für das erfindungsgemäße Verfahren nicht von relevanter Bedeutung, soweit im Rahmen des Verfahrens das zu reinigende Wasser auf eine höhere Temperatur erwärmt wird, oder einer Abkühlung des Wassers durch Wärmezufuhr entgegengewirkt werden soll. Es handelt sich bei dem Verfahren zur Wasserentsalzung aber um ein Verfahren, bei dem das Wasser durch Verdampfen von gelöstem Salz getrennt wird, nämlich eine Multistage Flash Evaporation (MFS) oder als Multieffekt-Destillation (MED) von denen insbesondere eine Multieffekt-Destillation zweckmäßig ist. Beides sind mehrstufige Verfahren, bei denen die eingespeiste Wärme auf jeweils abfallenden Temperatur- und Druckniveaus mehrfach genutzt werden kann und die insbesondere geeignet sind, auf niedrigem Temperaturniveau anfallende Prozesswärme zur Erzeugung von Frischwasser zu nutzen. Solche Verfahren erlauben es, auch niederwertige Prozesswärme, wie sie beispielsweise bei der Direktchlorierung von Ethylen anfällt, zur Gewinnung von Frischwasser aus Meerwasser zu nutzen.

Thermische Verfahren zur Entsalzung von Salz- und insbesondere Meerwasser haben in den letzten Jahren hinsichtlich ihrer Effektivität deutliche Verbesserungen erfahren. So werden beispielsweise in der WO 2015/154142 A1 Verfahren beschrieben, die die Zufuhr der für die Wasserverdampfung benötigten Energie auf einem sehr niedrigen Temperaturniveau erlauben. Diese auch als "boosted multieffect distillation" und "flash-boosted multieffect distillation" bezeichneten Verfahren sind im Kontext der hier beschriebenen Erfindung als Verfahren zur Wasserentsalzung, die im zweiten Anlagenteil erfolgt, besonders bevorzugt. Für eine detaillierte Beschreibung dieser Verfahren wird auf die WO 2015/154142 A1 verwiesen. Die Erfindung ist jedoch nicht auf die Verwendung eines Verfahrens nach WO 2015/154142 A1 beschränkt.

Wie vorstehend erwähnt ist es von besonderem Vorteil, dass in einer Wasserentsalzung generiertes Wasser für eine Chloralkalielektrolyse genutzt werden kann, wobei es, abhängig von der für die Wasserentsalzung verwendeten Technologie möglich ist, mehr als das für das Betreiben der Chloralkalielektrolyse benötigte Wasser in der Wasserentsalzung zu erzeugen. Demzufolge umfasst das erfindungsgemäße Verfahren in einer bevorzugten Ausführungsform einen dritten Anlagenteil, in dem eine Chlor-Alkali-Elektrolyse durchgeführt wird, wobei im zweiten Anlagenteil erzeugtes entsalztes Wasser mindestens anteilsmäßig für die Chlor-Alkali-Elektrolyse genutzt wird. Das in der Chlor-Alkali-Elektrolyse erzeugte Chlor wird bevorzugt mindestens anteilsmäßig in dem ersten Anlagenteil eingespeist, und dort mit Ethylen zu 1,2-Dichlorethan umgesetzt. Der Anlagenteil zur Umsetzung von Ethylen mit Chlor ist im erfindungsgemäßen Verfahren bevorzugt als Schlaufenreaktor ausgebildet, der ein Steigrohr, ein Ausgasgefäß und ein Fallrohr aufweist.

In einer weiteren Ausführungsform kann durch das erfindungsgemäße Verfahren Ansatzwasser für die Polymerisation von Vinylchlorid zu Polyvinylchlorid mittels thermischer Meerwasserentsalzung unter Verwendung von Reaktionswärme der Direktchlorierung bereitgestellt werden.

Ein Schlaufenreaktor kann, wie bei den Verfahren nach DE 19910964 A1 bzw. DE 102008020386 A1 aus einer flüssigkeitsgefüllten Schlaufe, die durch das Steigrohr, die Ausdampfungszone und das Fallrohr gebildet wird, bestehen. Im Steigrohr erfolgt die Einspeisung von Ethylen und die Zugabe von in 1,2-Dichlorethan gelöstem Chlor, wobei das Chlor zuvor, beispielsweise in einem Injektor, in flüssigem 1,2-Dichlorethan gelöst wurde. Letzteres wird bei dem beispielhaften Verfahren nach DE 19910964 A1 bzw. DE 102008020386 A1 aus dem Fallrohr aus der Reaktionsmischung abgezweigt und in einem Kühler auf eine niedrige Temperatur abgekühlt, um die Lösung des Chlors zu erleichtern. Das Ausgangsgefäß kann eine Abzugsvorrichtung für flüssiges 1,2-Dichlorethan und/oder eine Abzugsvorrichtung für gasförmiges 1,2-Dichlorethan aufweisen (meist und bevorzugt ist beides vorhanden). Die jeweiligen Zuführstellen und Abzugsvorrichtungen können aus praktischen Gründen auch mehrfach ausgeführt sein. Im Steigrohr der flüssigkeitsgefüllten Schlaufe reagieren Chlor und Ethylen miteinander zu siedendem 1,2-Dichlorethan, welches in der Ausdampfungszone zusammen mit nicht reagierten Ausgangsstoffen und inertem Begleitgas ausdampft.

Im Rahmen des erfindungsgemäßen Verfahrens wird bevorzugt die Wärme eines für die Umsetzung von Ethylen mit Chlor genutzten 1,2-Dichlorethanstroms und/oder Kondensationswärme eines an einem Reaktorkopf dampfförmig abgezogenen Produkts aus der Umsetzung für die Erhitzung von Wasser in der Entsalzung genutzt. D.h. Wärme des 1,2-Dichlorethanstroms wird genutzt, indem die 1,2-Dichlorethanreaktionsmischung aus dem Ausgasgefäß oder im Bereich des Fallrohrs aus dem Reaktor geführt und mit Hilfe eines Wärmetauschers die Wärme auf ein anderes, vorzugsweise flüssiges, Medium übertragen wird. Anschließend wird die 1,2-Dichlorethanreaktionsmischung im Bereich der Ausdampfungszone, im Bereich des Fallrohrs oder an einer Stelle des Steigrohrs vor Zuführungen für Ethylen wieder zugeführt. Kondensationswärme eines an einem Reaktorkopf dampfförmig abgezogenen Produkts wird ebenfalls zweckmäßig über einen Wärmetauscher auf ein vorzugsweise flüssiges Wärmetransfermedium übertragen, wobei 1,2-Dichlorethan aus der Reaktionsmischung kondensiert.

Die thermische Energie/Wärme aus der 1,2-Dichlorethanreaktion kann direkt in die Wasserentsalzung geführt werden, in dem die warmen Produkte aus der Reaktion in einem Wärmetauscher mit zu entsalzendem Wasser thermisch in Kontakt gebracht werden (Wärmetransfer ohne Wärmetransfermedium), oder es kann ein Wärmetransfermedium verwendet werden, das die Wärmeenergie von den Reaktionsprodukten der 1,2-Dichlorethanherstellung aufnimmt (z.B. in einem ersten Wärmetauscher) und von dem die Wärmeenergie anschließend auf das zu entsalzende Wasser (z.B. in einem zweiten Wärmetauscher, der meist Bestandteil des Anlagenteils zur Wasserentsalzung ist) überträgt. Aus Konstruktionsgründen ist dabei eine Wärmeübertragung mit Hilfe eines Wärmetransfermediums bevorzugt. Ein besonders kostengünstiges und aufgrund seiner hohen Wärmekapazität geeignetes Wärmetransfermedium ist Wasser, mit dem auch eine Abkühlung der Reaktionsmischung auf Temperaturen von deutlich unter 95°C möglich ist. Entsprechend wird Wasser als Wärmetransfermedium für die Übertragung der Wärme vom ersten zum zweiten Anlagenteil genutzt.

Da durch ein Abkühlen auf Temperaturen von unter 100°C und insbesondere unter 80°C eine gute Löslichkeit von Chlorgas in 1,2-Dichlorethan sichergestellt werden kann, ist es für das Verfahren bevorzugt, wenn mindestens ein Teil des 1,2-Dichlorethans durch die Übertragung von Wärme auf ein Wärmetransfermedium auf eine Temperatur im Bereich von 40 bis 90°C, bevorzugt 45 bis 85°C, bevorzugter 50 bis 80°C und noch weiter bevorzugt 50 bis 65°C gekühlt wird. Zweckmäßig wird in diesen Teil anschließend Chlorgas eingeleitet, bevorzugt über einen Injektor.

Im erfindungsgemäßen Verfahren wird das Kühlwasser, welches zum Abkühlen des zuvor genannten 1,2-Dichlorethans genutzt wird auf maximal 95°C, bevorzugter 90°C und noch bevorzugter maximal 85°C erwärmt.

In einem weiteren Aspekt betrifft die vorliegende Erfindung eine Verwendung von Wärme aus einem Verfahren zur Erzeugung von 1,2-Dichlorethan aus Ethylen und Chlor zum Erhitzung von in einer Wasserentsalzung zu behandelndem Wasser, wobei die Wärme mit Hilfe einer Transfervorrichtung von 1,2-Dichlorethan auf das Wasser übertragen wird. Für bevorzugte Ausgestaltungen dieser Verwendung kann auf die vorstehenden Ausführungen zum entsprechenden Verfahren verwiesen werden.

In einem noch weiteren Aspekt betrifft die vorliegende Erfindung eine integrierte Anlage zur Herstellung von 1,2-Dichlorethan und zur Wasserentsalzung, wobei die integrierte Anlage einen ersten Anlagenteil mit einem Reaktor zur Umsetzung von Chlor mit Ethylen zu 1,2-Dichlorethan, einen zweiten Anlagenteil zur Wasserentsalzung und eine Vorrichtung zum Transfer von Wärmeenergie zwischen beiden Anlagenteilen aufweist. Die Anlage ist bevorzugt zur Durchführung eines Verfahrens, wie es vorstehend beschrieben ist, angepasst bzw. ausgelegt.

Die Anlage ist eine integrierte Anlage, d.h. der erste und zweite Anlagenteil sind räumlich benachbart oder in räumlicher Nähe angeordnet, und es sind ein oder mehrere Leitungen vorhanden, mit denen Wärmeenergie vom ersten Anlagenteil zum zweiten Anlagenteil weitergeleitet werden kann.

In einer bevorzugten Ausführungsform weist die Anlage zusätzlich einen Anlagenteil zur Chloralkalielektrolyse auf, wobei der Anlagenteil zur Wasserentsalzung fließfähig mit dem Anlagenteil zur Chloralkalielektrolyse verbunden ist, um entsalztes Wasser von der Wasserentsalzung zur Chloralkalielektrolyse zuführen zu können. "Fließfähig verbunden" heißt hier, dass eine Leitung vorhanden ist, die einerseits mit der Produktseite der Wasserentsalzung und andererseits mit der Ausgangsmaterialseite der Chloralkalielektrolyse verbunden ist. In einer besonders bevorzugten Ausführungsform weist die Anlage zusätzliche eine Leitung auf, mit der in der Chloralkalielektrolyse hergestelltes Chlor (d.h. Chlorgas, Cl₂) in den Anlagenteil zur Herstellung von 1,2-Dichlorethan überführt werden kann.

In einer weiteren bevorzugten Ausführungsform weist die Anlage zusätzlich einen Anlagenteil zur Polymerisation von Vinylchlorid auf, wobei der Anlagenteil zur Wasserentsalzung fließfähig mit dem Anlagenteil zur Polymerisation von Vinylchlorid verbunden ist, um entsalztes Wasser von der Wasserentsalzung zur Polymerisation von Vinylchlorid zuführen zu können. "Fließfähig verbunden" heißt hier, dass eine Leitung vorhanden ist, die einerseits mit der Produktseite der Wasserentsalzung und andererseits mit der Ausgangsmaterialseite der Polymerisation von Vinylchlorid verbunden ist. In der Polymerisation von Vinylchlorid wird das Wasser zweckmäßig als Ansatzwasser verwendet.

In der beschriebenen Anlage ist der Anlagenteil zur Wasserentsalzung zweckmäßig als Multistage Flash Evaporation, Multieffekt-Destillation, boosted multieffect distillation oder flash-boosted multieffect distillation ausgebildet. Alternativ und bevorzugt zusätzlich dazu ist der Reaktor im Anlagenteil zur Umsetzung von Chlor mit Ethylen zu 1,2-Dichlorethan als Schlaufenreaktor ausgebildet. Darüber hinaus ist es bevorzugt, wenn die Anlage ein oder mehrere Wärmetauscher aufweist, der oder die über Leitungen mit dem Kopfraum eines Ausgasgefäßes des Schlaufenreaktors und mit dem Ausgasgefäß oder dem Fallrohr so verbunden ist, dass bei Betrieb des Reaktors flüssiges Material zum Wärmetauscher geführt werden kann. In einer ganz bevorzugten Ausführungsform ist die andere Seite des Wärmetauschers mit einem Kreislaufsystem für Wärmeübertragungsfluid verbunden, das seinerseits thermisch mit dem Anlagenteil zur Wasserentsalzung gekoppelt ist.

Im Folgenden wird die vorliegende Erfindung sowie deren Ausführungsformen anhand von Figuren näher illustriert:
Figur 1 zeigt eine beispielhafte Ausführungsform einer erfindungsgemäßen Anlage zur Direktchlorierung von Ethylen mit einer thermischen Meerwasser-Entsalzungsanlage.
Figur 2 zeigt eine beispielhafte, schematische Ausführungsform einer erfindungsgemäßen Anlagenkonfiguration.

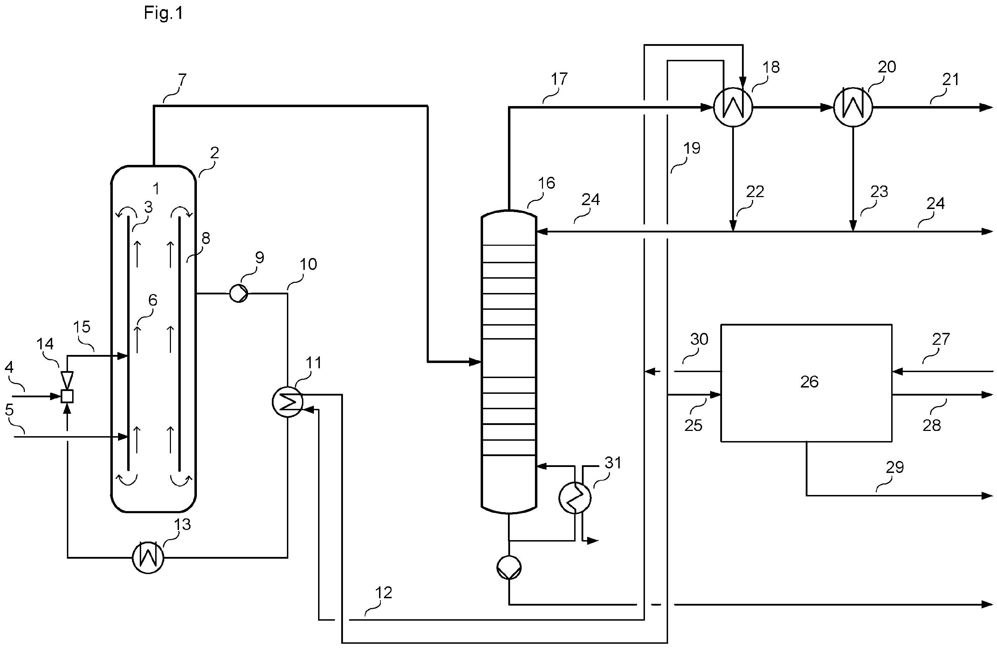

Figur 1 wird folgend näher erläutert:
In einem Schlaufenreaktor (1), bestehend aus einem Reaktionsbehälter (2) und einem inneren Steigrohr (3) werden Chlor (4) und Ethylen (5) in einem umlaufenden, flüssigen EDC-Strom (6) zu EDC umgesetzt. Im oberen Teil des Reaktors siedet das Reaktionsgemisch und das Produkt (7) wird dampfförmig aus dem Reaktor abgezogen. Ethylen wird im unteren Teil des Steigrohrs (3) über eine Verteilvorrichtung (nicht dargestellt) zugegeben und löst sich im umlaufenden EDC-Strom auf.

Aus dem Ringspalt des Reaktors (8) wird mittels einer EDC-Kreislaufpumpe (9) ein EDC-Teilstrom (10) entnommen und in einem ersten Kreislaufkühler (11) unter Anwärmung eines ersten Heisswasser-Teilstromes (12) abgekühlt. In einem zweiten EDC-Kreislaufkühler (13) wird der EDC-Teilstrom ggf. weiter auf die für die Verwendung in der Reaktion erforderliche Temperatur abgekühlt und dient in einer Strahlpumpe (14) zum Ansaugen und Lösen des Chlors (4). Der chlorhaltige EDC-Kreislaufstrom (15) wird nun über eine Verteilvorrichtung (nicht dargestellt) im Steigrohr (3) dem umlaufenden EDC-Strom zugegeben, der bereits gelöstes Ethylen enthält. Die Direktchlorierungsreaktion findet nun in der flüssigen Phase statt.

Im oberen Teil des Steigrohrs (3) beginnt das Reaktionsgemisch durch Abnahme des hydrostatischen Druckes zu sieden und verdampft teilweise. Dampfförmiges EDC (7) wird am Reaktorkopf abgezogen und in eine Destillationskolonne (16) zur Entfernung höher siedender Nebenprodukte eingespeist. Das reine Produkt (17) wir am Kopf der Kolonnen abgezogen und mittels eines Kopfkondensators (18) unter Anwärmen eines zweiten Heisswasser-Teilstromes (19) zum größten Teil kondensiert. Nachdem in mindestens einem Nachkondensator (20) weiteres EDC kondensiert wurde, wird der verbleibende Abgasstrom (21) zur weiteren Verarbeitung zur Anlagengrenze gegeben. Die kondensierten Produkt-EDC-Ströme (22), (23) werden teilweise als Rücklauf (24) auf die Kolonne gegeben. Die verbleibende EDC-Menge wird als Produkt zur Anlagengrenze gegeben. Die für die Destillation erforderliche externe Wärmemenge wird durch den Umlaufverdampfer (31) am Sumpf der Kolonne zugeführt.

Die vereinigten, vorgewärmten Wasserströme (12), (19) werden als Heisswasser-Vorlauf (25) einer mehrstufigen Anlage zur thermischem Meerwasserentsalzung (26) zugeführt. Seewasser (27) wird in die Entsalzungsanlage eingespeist, während ein konzentrierter Seewasserstrom (28) zum Meer zurückgegeben wird. Frischwasser (29) wird der weiteren Verwendung innerhalb oder außerhalb des Anlagenkomplexes zugeführt. Der Heisswasser-Rücklaufstrom (30) wird aufgeteilt und zur Erwärmung wieder den Wärmeüberträgern (11) und (18) zugeführt.

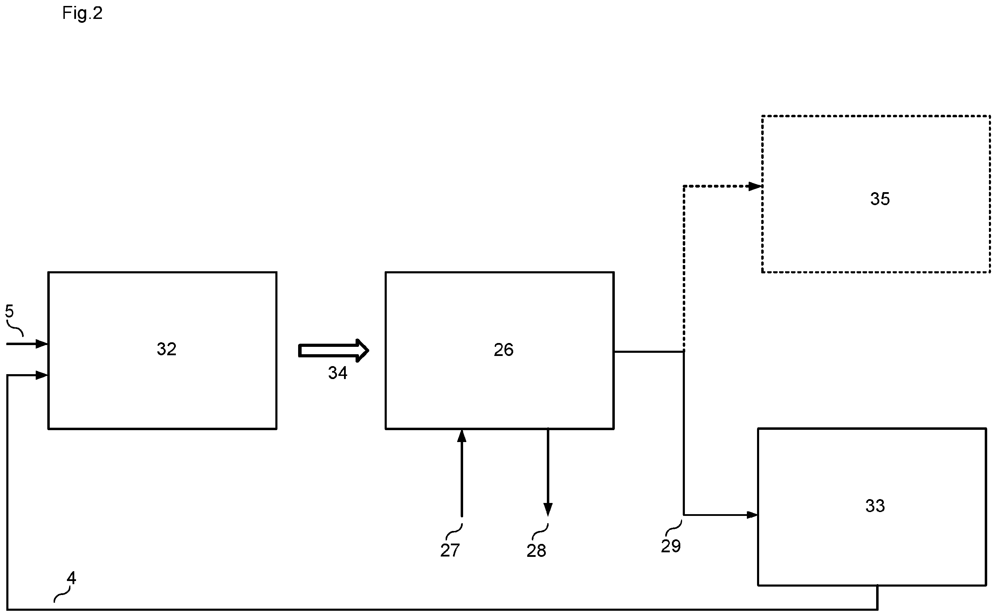

Figur 2 wird folgend näher erläutert:
Die im Beispiel genannten Material-und Wärmeströme beziehen sich auf eine Dichlorethan-Kapazität von ca. 327 kt/a 1,2-Dichlorethan, was in einer sog. Balanced-Anlage zur Herstellung von Vinylchlorid bzw. Polyvinylchlorid einer Vinylchlorid- bzw. Polyvinylchlorid-Kapazität von 400 kt/a entsprechen würde. Mengenströme werden nur genannt, soweit zur Erläuterung der Erfindung erforderlich.

In einer Direktchlorierungsanlage (32) werden Chlor (4) aus einer Chlor - Alkali-Elektrolyseanlage (33) und Ethylen (5) miteinander zu 1,2-Dichlorethan (nicht dargestellt) umgesetzt. Die Reaktionswärme entspricht einer thermischen Leistung von ca. 25 MW.

Aus der Direktchlorierung (32) werden ca. 18 MW an thermischer Leistung (34) ausgekoppelt und zur Beheizung einer Anlage zur thermischen Meerwasserentsalzung (26) verwendet. Dies entspricht einem Rückgewinnungsgrad von ca. 72 %. Hierfür wird der Meerwasserentsalzungsanlage Meerwasser (27) zugeführt und eingedampftes Meerwasser (28) wieder zur Anlagengrenze zurückgegeben.

Es lassen sich ca. 92 t/h (ca.2200 t/d) entsalztes Wasser (29) gewinnen. Der Bedarf an entsalztem Wasser der Chlor-Alkali-Elektrolyseanlage beträgt ca. 2100 t/d. Der Wasserbedarf der Chlor-Alkali-Elektrolyseanlage lässt sich also vollständig durch thermische Entsalzung von Meerwasser mit der Reaktionswärme der Direktchlorierung decken.

Alternativ (10) kann das entsalzte Wasser als Ansatzwasser in einer Anlage zur Herstellung von Polyvinylchlorid (35) verwendet werden. Der Wasserbedarf der Polyvinylchlorid-Anlage beträgt ca. 2760 t/d und kann zu ca. 76 % gedeckt werden.

Die Erfindung ist jedoch nicht auf die Beispiele gemäß Figuren 1 und 2 beschränkt. Insbesondere lässt sich durch erhöhten apparativen Aufwand noch ein höherer Anteil der Reaktionswärme der Direktchlorierung zurückgewinnen bzw. mehr entsalztes Wasser herstellen.

### Bezugszeichenliste

- 1: Schlaufenreaktor
- 2: Reaktionsgefäß
- 3: Steigrohr
- 4: Chlor
- 5: Ethylen
- 6: Umlaufender EDC-Strom
- 7: Produkt-EDC, dampfförmig
- 8: Ringspalt
- 9: EDC-Kreislaufpumpe
- 10: EDC-Teilstrom
- 11: EDC-Kreislaufkühler I
- 12: Heisswasser-Teilstrom I
- 13: EDC-Kreislaufkühler II
- 14: Strahlpumpe
- 15: Chlorhaltiger EDC-Kreislaufstrom
- 16: Hochsieder-Kolonne
- 17: Produkt-EDC, dampfförmig
- 18: Kopfkondensator
- 19: Heisswasser-Teilstrom II
- 20: Nachkondensator
- 21: Abgas
- 22: Produkt-EDC, flüssig
- 23: Produkt-EDC- flüssig
- 24: Rücklauf
- 25: Heißwasser-Vorlauf
- 26: Thermische Meerwasser-Entsalzungsanlage
- 27: Seewasser
- 28: Seewasser, Konzentrat
- 29: Frischwasser
- 30: Heisswasser-Rücklauf
- 31: Umlaufverdampfer
- 32: Direktchlorierungs-Anlage
- 33: Chlor-Alkali-Elektrolyse-Anlage
- 34: Reaktionswärme der Direktchlorierung
- 35: PVC-Anlage (optional)

## Patentansprüche

1. Verfahren zur Erzeugung von 1,2-Dichlorethan aus Ethylen und Chlor und zur Wasserentsalzung, wobei in einem ersten Anlagenteil Ethylen mit Chlor zu 1,2-Dichlorethan umgesetzt und in einem zweiten Anlagenteil eine Wasserentsalzung durchgeführt wird, **dadurch gekennzeichnet, dass** die bei der Umsetzung von Ethylen mit Chlor entstehende Wärme für eine Erhitzung von Wasser in der Entsalzung genutzt wird, wobei die Wasserentsalzung als Multistage Flash Evaporation oder als Multieffekt-Destillation, bevorzugt als Multieffekt-Destillation, durchgeführt wird, wobei Wasser als Wärmetransfermedium für die Übertragung der Wärme von ersten zum zweiten Anlagenteil genutzt wird und wobei das Wasser zum Abkühlen des 1,2-Dichlorethans auf maximal 95°C erwärmt wird.

2. Verfahren nach Anspruch 1, **dadurch gekennzeichnet, dass** die Wasserentsalzung als Meerwasserentsalzung durchgeführt wird.

3. Verfahren mindestens einem der Ansprüche 1 oder 2, **dadurch gekennzeichnet, dass** die bei der Umsetzung von Ethylen mit Chlor entstehende Wärme zur Beheizung eines thermischen Prozesses zur Wasserentsalzung mittels der boosted multieffect distillation oder der flash-boosted multieffect distillation genutzt wird.

4. Verfahren nach mindestens einem der vorangehenden Ansprüche, wobei das weiterhin einen dritten Anlagenteil umfasst, in dem eine Chlor-Alkali-Elektrolyse durchgeführt wird und wobei im zweiten Anlagenteil erzeugtes entsalztes Wasser mindestens anteilsmäßig für die Chlor-Alkali-Elektrolyse genutzt wird.

5. Verfahren nach mindestens einem der vorangehenden Ansprüche, **dadurch gekennzeichnet, dass** die Umsetzung von Ethylen mit Chlor zu 1,2-Dichlorethan in einem Schlaufenreaktor durchgeführt wird.

6. Verfahren nach mindestens einem der vorangehenden Ansprüche, **dadurch gekennzeichnet, dass** die Wärme eines für die Umsetzung von Ethylen mit Chlor genutzten 1,2-Dichlorethanstroms und/oder Kondensationswärme eines an einem Reaktorkopf dampfförmig abgezogenen Produkts aus der Umsetzung für die Erhitzung von Wasser in der Entsalzung genutzt wird.

7. Verfahren nach Anspruch 6, **dadurch gekennzeichnet, dass** mindestens ein Teil des 1,2-Dichlorethans durch die Übertragung von Wärme auf ein Wärmetransfermedium auf eine Temperatur im Bereich von 40 bis 90°C und bevorzugt 50 bis 65°C gekühlt wird.

8. Verwendung von Wärme aus einem Verfahren zur Erzeugung von 1,2-Dichlorethan aus Ethylen und Chlor zur Erhitzung von in einer Wasserentsalzung zu behandelndem Wasser, wobei die Wärme mit Hilfe einer Transfervorrichtung von 1,2-Dichlorethan auf das Wasser übertragen wird, wobei die bei der Umsetzung von Ethylen mit Chlor entstehende Wärme zur Beheizung eines thermischen Prozesses zur Wasserentsalzung mittels der boosted multieffect distillation oder der flash-boosted multieffect distillation genutzt wird, wobei Wasser als Wärmetransfermedium für die Übertragung der Wärme von ersten zum zweiten Anlagenteil genutzt wird und wobei das Wasser zum Abkühlen des 1,2-Dichlorethans auf maximal 95°C erwärmt wird.

9. Anlage zur Herstellung von 1,2-Dichlorethan und zur Wasserentsalzung, bevorzugt zur Durchführung eines Verfahrens gemäß einem der Ansprüche 1 bis 7, wobei die Anlage einen ersten Anlagenteil mit einem Reaktor zur Umsetzung von Chlor mit Ethylen zu 1,2-Dichlorethan, einen zweiten Anlagenteil zur Wasserentsalzung und eine Vorrichtung zur Transfer von Wärmeenergie zwischen beiden Anlagenteilen aufweist, wobei der Anlagenteil zur Wasserentsalzung als boosted multieffect distillation oder flash-boosted multieffect distillation ausgestaltet ist und die Anlage so ausgelegt ist, dass Transfer von Wärmeenergie zwischen dem ersten und zweiten Anlagenteils mittels Wasser als Wärmetransfermediums möglich ist, und der zweite Anlagenteil zur Wasserentsalzung für einen Betrieb mit Wasser als Wärmetransfermedium bei einer Temperatur von maximal 95 °C ausgelegt ist.

10. Anlage gemäß Anspruch 9, wobei die Anlage zusätzlichen einen Anlagenteil zur Chloralkalielektrolyse umfasst, und wobei der Anlagenteil zur Wasserentsalzung fließfähig mit dem Anlagenteil zur Chloralkalielektrolyse verbunden ist, um entsalztes Wasser von der Wasserentsalzung zur Chloralkalielektrolyse zuführen zu können.

11. Anlage gemäß Anspruch 91 oder 10, wobei der Anlagenteil zur Wasserentsalzung als Multistage Flash Evaporation, Multieffekt-Destillation boosted multieffect distillation oder flash-boosted multieffect distillation ausgebildet ist.

12. Anlage gemäß mindestens einem der Ansprüche 9 bis 11, wobei der Reaktor im Anlagenteil zur Umsetzung von Chlor mit Ethylen zu 1,2-Dichlorethan als Schlaufenreaktor ausgebildet ist.

## Claims

1. A method for generation of 1,2-dichloroethane from ethylene and chlorine and for water desalination, by reacting ethylene with chlorine to 1,2-dichloroethane in a first plant part and performing a water desalination in a second plant part, **characterized in that** the heat produced in the reaction of ethylene with chlorine is utilized for heating of water in the desalination, wherein the water desalination is performed as multistage flash evaporation or as multieffect distillation, preferably as multieffect distillation, wherein water is utilized as heat transfer medium for transfer of the heat from the first to the second plant part, and wherein the water for cooling of the 1,2-dichloroethane is heated to not more than 95°C.

2. The method as claimed in claim 1, **characterized in that** the water desalination is performed as seawater desalination.

3. The method as claimed in at least one of claims 1 or 2, **characterized in that** the water produced in the reaction of ethylene with chlorine is utilized for heating of a thermal process for water desalination via boosted multieffect distillation or flash-boosted multieffect distillation.

4. The method as claimed in at least one of the preceding claims, further comprising a third plant part, in which a chloralkali process is performed and where desalinated water generated in the second plant part is utilized at least fractionally for the chloralkali process.

5. The method as claimed in at least one of the preceding claims, **characterized in that** the reaction of ethylene with chlorine to 1,2-dichloroethane is performed in a loop reactor.

6. The method as claimed in at least one of the preceding claims, **characterized in that** the heat of a 1,2-dichloroethane stream utilized for reaction of ethylene with chlorine and/or heat of condensation from a product from the reaction that is drawn off in vapor form at a reactor top is utilized for heating of water in the desalination.

7. The method as claimed in claim 6, **characterized in that** at least part of the 1,2-dichloroethane is cooled, by transfer of heat to a heat transfer medium, to a temperature in the range from 40 to 90°C and preferably 50 to 65°C.

8. The use of heat from a method for generation of 1,2-dichloroethane from ethylene and chlorine for heating of water to be treated in a water desalination, wherein the heat is transferred from 1,2-dichloroethane to the water by means of a transfer apparatus, wherein the heat produced in the reaction of ethylene with chlorine is utilized for heating of a thermal process for water desalination via boosted multieffect distillation or flash-boosted multieffect distillation, wherein water is utilized as heat transfer medium for transfer of the heat from the first to the second plant part, and wherein the water for cooling of the 1,2-dichloroethane is heated to not more than 95°C.

9. A plant for production of 1,2-dichloroethane and for water desalination, preferably for performance of a method as claimed in any of claims 1 to 7, wherein the plant comprises a first plant part with a reactor for reaction of chlorine with ethylene to 1,2-dichloroethane, a second plant part for water desalination, and an apparatus for transfer of heat energy between the two plant parts, wherein the plant part for water desalination is designed as a boosted multieffect distillation or flash-boosted multieffect distillation and the plant is configured such that transfer of heat energy between the first and second plant part is possible via water as heat transfer medium, and the second plant part for water desalination is configured for operation with water as heat transfer medium at a temperature of not more than 95°C.

10. The plant as claimed in claim 9, wherein the plant additionally comprises a plant part for the chloralkali process, and wherein the plant part for water desalination is fluidly connected to the plant part for the chloralkali process, to allow desalinated water from the water desalination to be supplied to the chloralkali process.

11. The plant as claimed in claim 9 or 10, wherein the plant part for water desalination is embodied as a multistage flash evaporation, multieffect distillation, boosted multieffect distillation or flash-boosted multieffect distillation.

12. The plant as claimed in at least one of claims 9 to 11, wherein the reactor, in the plant part for reaction of chlorine with ethylene to 1,2-dichloroethane, is embodied as a loop reactor.

## Revendications

1. Procédé de production de 1,2-dichloroéthane à partir d'éthylène et de chlore et de dessalement de l'eau, par réaction de l'éthylène avec le chlore pour former du 1,2-dichloroéthane dans une première partie d'installation et réalisation d'un dessalement de l'eau dans une seconde partie d'installation, **caractérisé en ce que** la chaleur produite lors de la réaction de l'éthylène avec le chlore est utilisée pour le chauffage de l'eau dans le dessalement, le dessalement de l'eau étant réalisé sous forme d'une évaporation instantanée à plusieurs étages ou d'une distillation à effets multiples, de préférence d'une distillation à effets multiples, l'eau étant utilisée comme fluide caloporteur pour le transfert de la chaleur de la première à la seconde partie d'installation, et l'eau destinée au refroidissement du 1,2-dichloroéthane étant chauffée à une température ne dépassant pas 95°C.

2. Procédé selon la revendication 1, **caractérisé en ce que** le dessalement de l'eau est réalisé sous forme d'un dessalement de l'eau de mer.

3. Procédé selon au moins l'une des revendications 1 ou 2, **caractérisé en ce que** l'eau produite lors de la réaction de l'éthylène avec le chlore est utilisée pour le chauffage d'un procédé thermique de dessalement de l'eau par distillation à effets multiples assistée ou par distillation à effets multiples assistée par évaporation instantanée.

4. Procédé selon au moins l'une des revendications précédentes, comprenant en outre une troisième partie d'installation, dans laquelle un procédé chlore-soude est réalisé et dans laquelle l'eau dessalée produite dans la seconde partie d'installation est utilisée au moins partiellement pour le procédé chlore-soude.

5. Procédé selon au moins l'une des revendications précédentes, **caractérisé en ce que** la réaction de l'éthylène avec le chlore pour former du 1,2-dichloroéthane est réalisée dans un réacteur à boucle.

6. Procédé selon au moins l'une des revendications précédentes, **caractérisé en ce que** la chaleur d'un courant de 1,2-dichloroéthane utilisé pour la réaction de l'éthylène avec le chlore et/ou la chaleur de condensation d'un produit issu de la réaction soutiré sous forme de vapeur à la partie supérieure d'un réacteur est utilisée pour le chauffage de l'eau dans le dessalement.

7. Procédé selon la revendication 6, **caractérisé en ce qu'**au moins une partie du 1,2-dichloroéthane est refroidie, par transfert de chaleur vers un fluide caloporteur, à une température comprise entre 40 et 90°C et de préférence entre 50 et 65°C.

8. Utilisation de la chaleur provenant d'un procédé de production de 1,2-dichloroéthane à partir d'éthylène et de chlore pour le chauffage de l'eau à traiter dans un dessalement de l'eau, la chaleur étant transférée du 1,2-dichloroéthane vers l'eau au moyen d'un appareil de transfert, la chaleur produite lors de la réaction de l'éthylène avec le chlore étant utilisée pour le chauffage d'un procédé thermique de dessalement de l'eau par distillation à effets multiples assistée ou par distillation à effets multiples assistée par évaporation instantanée, l'eau étant utilisée comme fluide caloporteur pour le transfert de la chaleur de la première à la seconde partie d'installation, et l'eau destinée au refroidissement du 1,2-dichloroéthane étant chauffée à une température ne dépassant pas 95°C.

9. Installation de production de 1,2-dichloroéthane et de dessalement de l'eau, de préférence pour la mise en œuvre d'un procédé selon l'une quelconque des revendications 1 à 7, l'installation comprenant une première partie d'installation comportant un réacteur pour la réaction du chlore avec l'éthylène afin de former du 1,2-dichloroéthane, une seconde partie d'installation pour le dessalement de l'eau, et un appareil destiné au transfert d'énergie thermique entre les deux parties d'installation, la partie d'installation pour le dessalement de l'eau étant conçue sous forme d'une distillation à effets multiples assistée ou d'une distillation à effets multiples assistée par évaporation instantanée et l'installation étant configurée de telle sorte qu'un transfert d'énergie thermique entre la première et la seconde partie d'installation soit possible par l'intermédiaire de l'eau en tant que fluide caloporteur, et la seconde partie d'installation pour le dessalement de l'eau étant configurée pour fonctionner avec de l'eau en tant que fluide caloporteur à une température ne dépassant pas 95°C.

10. Installation selon la revendication 9, dans laquelle l'installation comprend en outre une partie d'installation pour le procédé chlore-soude, et dans laquelle la partie d'installation pour le dessalement de l'eau est reliée par voie fluidique à la partie d'installation pour le procédé chlore-soude, afin de permettre l'alimentation du procédé chlore-soude en eau dessalée provenant du dessalement de l'eau.

11. Installation selon la revendication 9 ou 10, dans laquelle la partie d'installation pour le dessalement de l'eau est réalisée sous forme d'une évaporation instantanée à plusieurs étages, d'une distillation à effets multiples, d'une distillation à effets multiples assistée ou d'une distillation à effets multiples assistée par évaporation instantanée.

12. Installation selon au moins l'une des revendications 9 à 11, dans laquelle le réacteur, dans la partie d'installation destinée à la réaction du chlore avec l'éthylène pour former du 1,2-dichloroéthane, est réalisé sous forme d'un réacteur à boucle.
